(19) **Europäisches Patentamt / European Patent Office / Office européen des brevets**

(11) **EP 3 384 854 A1**

(12) # EUROPEAN PATENT APPLICATION
published in accordance with Art. 153(4) EPC

(43) Date of publication:
**10.10.2018 Bulletin 2018/41**

(21) Application number: **16870449.2**

(22) Date of filing: **16.11.2016**

(51) Int Cl.:
**A61B 8/14** *(2006.01)*     **A61B 8/12** *(2006.01)*

(86) International application number:
**PCT/JP2016/084003**

(87) International publication number:
**WO 2017/094511 (08.06.2017 Gazette 2017/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.11.2015 JP 2015233490**

(71) Applicant: **Olympus Corporation**
**Hachioji-shi, Tokyo 192-8507 (JP)**

(72) Inventor: **KOZAI, Shigenori**
**Hachioji-shi**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ULTRASONIC OBSERVATION DEVICE, OPERATION METHOD FOR ULTRASONIC OBSERVATION DEVICE, AND OPERATION PROGRAM FOR ULTRASONIC OBSERVATION DEVICE**

(57)     An ultrasound observation device according to the present invention is an ultrasound observation device configured to generate an ultrasound image based on an ultrasound signal acquired by an ultrasound probe including an ultrasound transducer that transmits ultrasound to an observation target and receives ultrasound reflected from the observation target. The ultrasound observation device includes: a frequency analysis unit configured to analyze a frequency of a signal generated based on the ultrasound signal to calculate a plurality of frequency spectra; a comparison unit configured to compare a physical quantity based on the ultrasound reflected from the observation target with a threshold set according to the physical quantity; and a feature calculation unit configured to calculate a frequency feature based on the frequency spectrum calculated by the frequency analysis unit and a comparison result obtained by the comparison unit.

FIG.1

EP 3 384 854 A1

**Description**

Field

The present invention relates to an ultrasound observation device that observes a tissue of an observation target using ultrasound, a method of operating the ultrasound observation device, and a program for operating the ultrasound observation device.

Background

Conventionally, in an ultrasound observation device that observes an observation target tissue using ultrasound, a technique of calculating a feature of a frequency spectrum of an ultrasound signal having characteristics corresponding to tissue characteristics and generating a feature image for identifying the tissue characteristics on the basis of the feature is known. In this technique, after the frequency of the received ultrasound signal is analyzed to acquire a frequency spectrum, an approximate expression of the frequency spectrum in a predetermined frequency band is calculated and a feature is extracted from the approximate expression.

When a feature is extracted, it may not be possible to obtain an accurate feature in a noise region which is a low echo region due to the influence of noise. As a technique of determining a noise region, an ultrasound diagnosis device that identifies a noise region as a low S/N region and displays information on the low S/N region together with an attenuation image (a feature image) which is an image based on an attenuation factor is known (for example, see Patent Literature 1). In this technique, it is determined whether each of predetermined regions corresponds to a low S/N region and a determination result is displayed as the information on the low S/N region. In this way, an operator such as a physician can determine whether a position being analyzed corresponds to a noise region.

Citation List

Patent Literature

Patent Literature 1: JP 2013-005876 A

Summary

Technical Problem

The technique disclosed in Patent Literature 1 determines whether each of predetermined regions corresponds to a low S/N region and displays the determination result. However, a feature image is not generated on the basis of the determination result. Due to this, the determination result is not reflected on a feature image and a feature of an observation target is not calculated appropriately.

The present invention has been realized in view of the above-described circumstances and an object thereof is to provide an ultrasound observation device, a method of operating the ultrasound observation device, and a program for operating the ultrasound observation device capable of calculating a feature appropriately even when a noise region is included.

Solution to Problem

To solve the problem described above and to achieve the object, an ultrasound observation device according to the present invention is an ultrasound observation device configured to generate an ultrasound image based on an ultrasound signal acquired by an ultrasound probe including an ultrasound transducer that transmits ultrasound to an observation target and receives ultrasound reflected from the observation target. The ultrasound observation device includes: a frequency analysis unit configured to analyze a frequency of a signal generated based on the ultrasound signal to calculate a plurality of frequency spectra; a comparison unit configured to compare a physical quantity based on the ultrasound reflected from the observation target with a threshold set according to the physical quantity; and a feature calculation unit configured to calculate a frequency feature based on the frequency spectrum calculated by the frequency analysis unit and a comparison result obtained by the comparison unit.

In the ultrasound observation device according to the present invention, the comparison unit is configured to compare the physical quantity with the threshold for each of a plurality of regions set in the ultrasound image, and the feature calculation unit is configured to set an attenuation factor according to the comparison result obtained by the comparison unit to calculate the frequency feature for each of the regions.

In the ultrasound observation device according to the present invention, the physical quantity is one selected from the group consisting of the frequency feature, a luminance of the ultrasound image, a change in an elastography, and a sound velocity.

In the ultrasound observation device according to the present invention, the physical quantity is related to the frequency feature.

In the ultrasound observation device according to the present invention, the physical quantity is a mid-band fit which is a value of a linear equation in a mid-frequency of a predetermined frequency band in the frequency spectrum, the mid-band fit being calculated by the feature calculation unit, the frequency band being approximated to the linear equation.

In the ultrasound observation device according to the present invention, the comparison unit is configured to determine whether a low echo region is included based on the physical quantity and the threshold, and the feature calculation unit is configured to calculate a frequency feature using a predetermined attenuation factor when a determination result obtained by the comparison unit indicates that the low echo region is included.

In the ultrasound observation device according to the present invention, the frequency analysis unit is configured to calculate a plurality of frequency spectra, and when the determination result obtained by the comparison unit indicates that the low echo region is not included, the feature calculation unit is configured to: calculate features of the plurality of frequency spectra; calculate a corrected feature of each of the frequency spectra by performing attenuation correction for eliminating an influence of attenuation of the ultrasound with respect to the feature of each of the frequency spectra for each of a plurality of attenuation factor candidate values that give different attenuation characteristics when the ultrasound propagates through the observation target; and set an optimal attenuation factor optimal for the observation target among the plurality of attenuation factor candidate values using the corrected feature.

In the ultrasound observation device according to the present invention, the feature calculation unit is configured to: calculate the feature by approximating each of the frequency spectra to an n-th order equation (n is a positive integer); calculate a statistical variation of the corrected feature for each of the attenuation factor candidate values; and set an attenuation factor candidate value of which the statistical variation is the smallest as the optimal attenuation factor.

In the ultrasound observation device according to the present invention, the feature calculation unit is configured to: approximate a predetermined frequency band in the frequency spectrum to a linear equation; calculate one or a plurality of features among an intercept and a slope of the linear equation and a mid-band fit which is a value of the linear equation at a mid-frequency of the frequency band, the one or plurality of features including any one of the slope and the mid-band fit; and set the optimal attenuation factor based on any one of the slope and the mid-band fit.

In the ultrasound observation device according to the present invention, the feature calculation unit is configured to: set the optimal attenuation factor based on the slope when the slope is calculated as the feature; and set the optimal attenuation factor based on the mid-band fit when the mid-band fit is calculated as the feature.

In the ultrasound observation device according to the present invention, the feature calculation unit is configured to: obtain the statistical variation as a function of the attenuation factor candidate value; and set an attenuation factor candidate value for which the statistical variation in the function is the smallest as the optimal attenuation factor.

The ultrasound observation device according to the present invention further includes: a feature image data generation unit configured to generate feature image data for displaying the frequency feature calculated by the feature calculation unit together with the ultrasound image in correlation with visual information.

A method of operating an ultrasound observation device according to the present invention is a method of operating an ultrasound observation device configured to generate an ultrasound image based on an ultrasound signal acquired by an ultrasound probe including an ultrasound transducer that transmits ultrasound to an observation target and receives ultrasound reflected from the observation target. The method includes: a frequency analysis step of causing a frequency analysis unit to analyze a frequency of a signal generated based on the ultrasound signal to calculate a plurality of frequency spectra; a comparison step of causing a comparison unit to compare a physical quantity based on the ultrasound reflected from the observation target with a threshold set according to the physical quantity; and a feature calculation step of causing a feature calculation unit to calculate a frequency feature based on the frequency spectrum calculated by the frequency analysis unit and a comparison result obtained by the comparison unit.

A program for operating an ultrasound observation device according to the present invention is a program for operating an ultrasound observation device configured to generate an ultrasound image based on an ultrasound signal acquired by an ultrasound probe including an ultrasound transducer that transmits ultrasound to an observation target and receives ultrasound reflected from the observation target. The program causes the ultrasound observation device to execute: a frequency analysis process of causing a frequency analysis unit to analyze a frequency of a signal generated based on the ultrasound signal to calculate a plurality of frequency spectra; a comparison process of causing a comparison unit to compare a physical quantity based on the ultrasound reflected from the observation target with a threshold set according to the physical quantity; and a feature calculation process of causing a feature calculation unit to calculate a frequency feature based on the frequency spectrum calculated by the frequency analysis unit and a comparison result obtained by the comparison unit.

Advantageous Effects of Invention

According to the present invention, it is possible to calculate a feature appropriately even when a noise region is included.

Brief Description of Drawings

FIG. 1 is a block diagram illustrating a configuration of an ultrasound observation system having an ultrasound observation device according to an embodiment of the present invention.

FIG. 2 is a diagram illustrating a relationship between a reception depth and an amplification factor in an amplification process performed by a signal amplification unit of the ultrasound observation device according to an embodiment of the present invention.

FIG. 3 is a diagram illustrating a relationship between a reception depth and an amplification factor in an amplification correction process performed by an amplification correction unit of the ultrasound observation device according to an embodiment of the present invention.

FIG. 4 is a diagram schematically illustrating a data arrangement in one sound ray of an ultrasound signal.

FIG. 5 is a diagram illustrating an example of a frequency spectrum calculated by a frequency analysis unit of the ultrasound observation device according to an embodiment of the present invention.

FIG. 6 is a diagram illustrating a straight line having a corrected feature calculated by an attenuation correction unit of the ultrasound observation device according to an embodiment of the present invention as a parameter.

FIG. 7 is a diagram schematically illustrating a distribution example of corrected features attenuated and corrected for the same observation target on the basis of two different attenuation factor candidate values.

FIG. 8 is a diagram for describing a region of interest set by the ultrasound observation device according to an embodiment of the present invention and division regions obtained by dividing the region of interest.

FIG. 9 is a flowchart illustrating an overview of a process performed by the ultrasound observation device according to an embodiment of the present invention.

FIG. 10 is a flowchart illustrating an overview of a process executed by a frequency analysis unit of the ultrasound observation device according to an embodiment of the present invention.

FIG. 11 is a diagram illustrating an overview of a process performed by an optimal attenuation factor setting unit of the ultrasound observation device according to an embodiment of the present invention.

FIG. 12 is a diagram schematically illustrating a display example of a feature image on a display device of the ultrasound observation system according to an embodiment of the present invention.

Description of Embodiments

Hereinafter, modes (hereinafter referred to as "embodiments") for carrying out the invention will be described with reference to the accompanying drawings.

(First Embodiment)

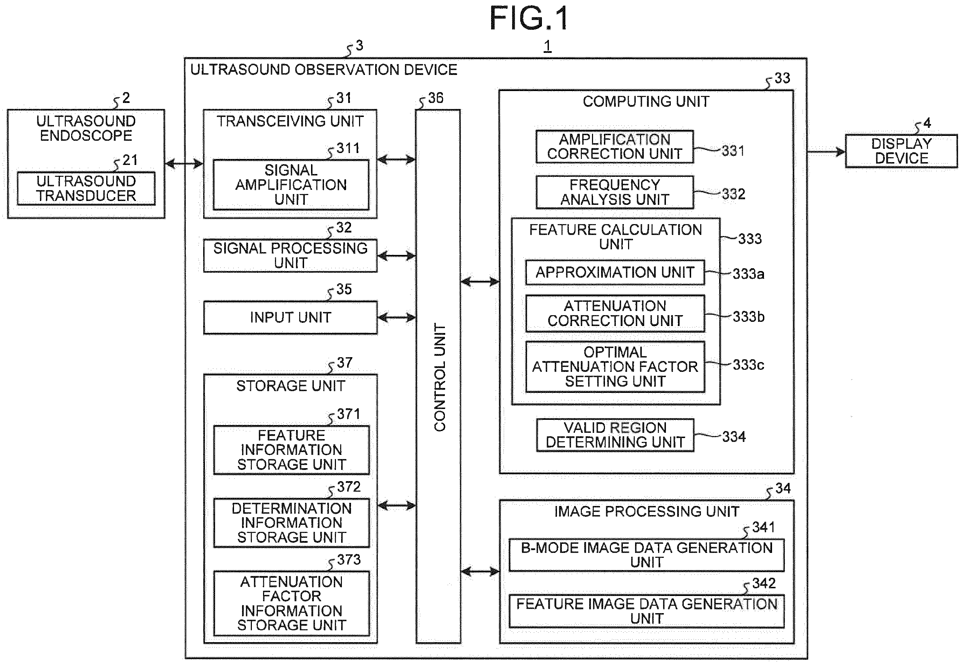

FIG. 1 is a diagram illustrating a configuration of an ultrasound observation system 1 having an ultrasound observation device 3 according to a first embodiment of the present invention. The ultrasound observation system 1 illustrated in the drawing includes an ultrasound endoscope 2 (an ultrasound probe) that transmits ultrasound to a subject which is an observation target and receives ultrasound reflected from the subject, an ultrasound observation device 3 that generates an ultrasound image on the basis of an ultrasound signal acquired by the ultrasound endoscope 2, and a display device 4 that displays the ultrasound image generated by the ultrasound observation device 3.

The ultrasound endoscope 2 has an ultrasound transducer 21 provided at a distal end thereof so as to convert an electric pulse signal received from the ultrasound observation device 3 to an ultrasound pulse (an acoustic pulse) to radiate the ultrasound pulse to a subject and convert an ultrasound echo reflected from the subject to an electric echo signal that represents the reflected ultrasound echo as a change in voltage to output the electric echo signal. The ultrasound transducer 21 may be a convex oscillator, a linear oscillator, or a radial oscillator. The ultrasound endoscope 2 may be configured such that the ultrasound transducer 21 performs scanning mechanically and may be configured such that a plurality of elements are provided in an array as the ultrasound transducer 21 and the elements associated with transmission and reception are electronically switched or the transmission and reception of the respective elements are delayed whereby the ultrasound transducer 21 performs scanning electronically.

The ultrasound endoscope 2 generally includes an imaging optical system and an imaging device. The ultrasound endoscope 2 can be inserted into a digestive tract (esophagus, stomach, duodenum, large intestine) or a respiratory organ (trachea/bronchus) of a subject and may capture the images of the digestive tract, the respiratory organ and surrounding organs (pancreas, gallbladder, bile duct, biliary tract, lymph node, mediastinum, blood vessels, or the like). Moreover, the ultrasound endoscope 2 includes a light guide that guides illumination light radiated to the subject during imaging. The light guide has a distal end reaching a distal end of an insertion portion of the ultrasound endoscope 2 inserted into the subject and a proximal end being connected to a light source device that generates illumination light. Without being limited to the ultrasound endoscope 2, an ultrasound probe that does not have an imaging optical system and an imaging device may be used.

The ultrasound observation device 3 further includes a transceiving unit 31 electrically connected to the ultrasound endoscope 2 to transmit a transmission signal (a pulse signal) made up of a high voltage pulse to the ultrasound transducer 21 on the basis of a predetermined waveform and a transmission timing and receive an echo signal which is an electric reception signal from the ultrasound transducer 21 to generate and output digital radio frequency (RF) signal data (hereinafter referred to as RF data), a signal processing unit 32 that generates digital B-mode reception data on the basis of the RF data received from the transceiving unit 31, a computing unit 33 that performs predetermined computation on the RF data received from the transceiving unit 31, an image processing unit 34 that generates various pieces of image data, an input unit 35 that is realized using a user interface such as a keyboard, a mouse, or a touch panel to receive input of various pieces of information, a control unit 36 that controls the entire ultrasound observation system 1, and a storage unit 37 that stores various pieces of information necessary for the operation of the ultrasound observation device 3.

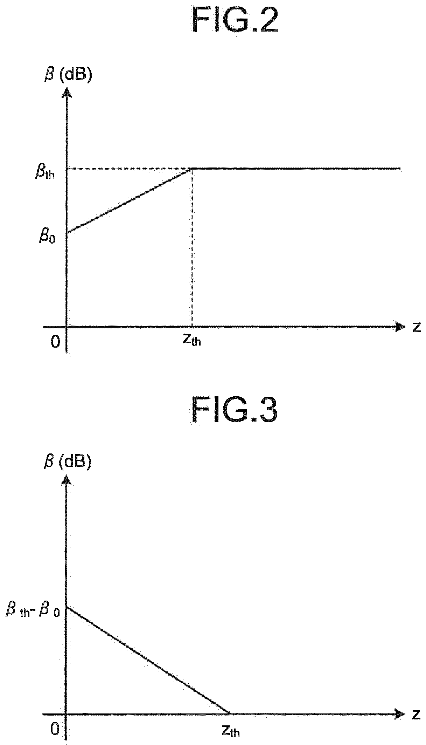

The transceiving unit 31 has a signal amplification unit 311 that amplifies an echo signal. The signal amplification unit 311 performs sensitivity time control (STC) such that the larger the reception depth of an echo signal, the higher the amplification factor with which the echo signal is amplified. FIG. 2 is a diagram illustrating a relationship between a reception depth and an amplification factor in the amplification process performed by the signal amplification unit 311. A reception depth z illustrated in FIG. 2 is an amount calculated on the basis of the time elapsed from a time point at which reception of ultrasound starts. As illustrated in FIG. 2, an amplification factor $\beta$ (dB) increases from $\beta_0$ to $\beta_{th}$ ($> \beta_0$) as the reception depth z increases when the reception depth z is smaller than a threshold $z_{th}$. Moreover, the amplification factor $\beta$ (dB) has a constant value $\beta_{th}$ when the reception depth z is equal to or larger than a threshold $z_{th0}$. The value of the threshold $z_{th}$ is set such that an ultrasound signal received from an observation target is almost attenuated and noise becomes dominant. More generally, the amplification factor $\beta$ may increase monotonically as the reception depth z increases when the reception depth z is smaller than the threshold $z_{th}$. The relationship illustrated in FIG. 2 is stored in advance in the storage unit 37.

The transceiving unit 31 generates RF data in a time domain by performing processing such as filtering on the echo signal amplified by the signal amplification unit 311 and then A/D converting the processed echo signal and outputs the generated RF data to the signal processing unit 32, the computing unit 33, and the storage unit 37. When the ultrasound endoscope 2 has a configuration in which the ultrasound transducer 21 having a plurality of elements arranged in an array performs scanning electronically, the transceiving unit 31 has a multi-channel circuit for beam synthesis corresponding to the plurality of elements.

A frequency band of the pulse signal transmitted by the transceiving unit 31 may be a wide band that covers an approximately entire linear-response frequency band for electro-acoustic conversion from a pulse signal to an ultrasound pulse by the ultrasound transducer 21. Moreover, various processing frequency bands of the echo signal in the signal amplification unit 311 may be a wide band that covers an approximately entire linear-response frequency band for acoustic-electric conversion from an ultrasound echo to an echo signal by the ultrasound transducer 21. Due to this, when a frequency spectrum approximation process to be described later is executed, it is possible to perform approximation with high accuracy.

The transceiving unit 31 also has a function of transmitting various control signals output by the control unit 36 to the ultrasound endoscope 2 and receiving various pieces of information including an identification ID from the ultrasound endoscope 2 to transmit the information to the control unit 36.

The signal processing unit 32 performs known processes such as band-pass filtering, envelope detection, and logarithmic conversion with respect to RF data to generate digital B-mode reception data. The logarithmic conversion involves taking a common logarithm of a quantity obtained by dividing RF data by a reference voltage $V_c$ to express the RF data as a decibel value. In the B-mode reception data, an amplitude or an intensity of a reception signal indicating the reflection strength of an ultrasound pulse is arranged in a transceiving direction (a depth direction) of the ultrasound pulse. The signal processing unit 32 outputs the generated B-mode reception data to the image processing unit 34. The signal processing unit 32 is realized using a general purpose processor such as a central processing unit (CPU) or a specific purpose integrated circuit that executes a specific function such as an application specific integrated circuit (ASIC) or a field programmable gate array (FPGA).

The computing unit 33 includes an amplification correction unit 331 that performs amplification correction with respect to the RF data generated by the transceiving unit 31 so that the amplification factor $\beta$ is constant regardless of a reception depth, a frequency analysis unit 332 that performs fast fourier transform (FFT) with respect to the amplification-corrected RF data to perform frequency analysis to thereby calculate a frequency spectrum, a feature calculation unit 333 that calculates a feature of the frequency spectrum, and a valid region determining unit 334 that determines whether a target region is a region that does not include a noise region and is a region (a valid region) valid for generating a feature image on the basis of the feature calculated by the feature calculation unit 333. The computing unit 33 is realized using a CPU and various computation circuits.

FIG. 3 is a diagram illustrating a relationship between a reception depth and an amplification factor in an amplification correction process performed by the amplification correction unit 331. As illustrated in FIG. 3, the amplification factor $\beta$ (dB) in the amplification process performed by the amplification correction unit 331 has a maximum value $\beta_{th} - \beta_0$ when the reception depth z is zero and decreases linearly until the reception depth z reaches the threshold $z_{th}$ from zero to become zero when the reception depth z is equal to or larger than the threshold $z_{th}$. The amplification correction unit 331 performs amplification correction with respect to a digital RF signal using the amplification factor determined in this manner whereby it is possible to cancel the influence of STC correction of the signal processing unit 32 and to output a signal of a constant amplification factor $\beta_{th}$. Naturally, the relationship between the reception depth z and the amplification factor $\beta$ in the amplification correction unit 331 is different depending on a relationship between the reception depth and the amplification factor in the signal processing unit 32.

The reasons for performing such amplification correction will be described. STC correction is a correction process of eliminating the influence of attenuation from an amplitude of an analog signal waveform by amplifying the amplitude of the analog signal waveform by an amplification factor that is uniform over an entire frequency band and increases monotonically in relation to the depth. Due to this, when a B-mode image used for converting an amplitude of an echo signal to a luminance and displaying the amplitude is generated, and a uniform tissue is scanned, the luminance value is constant regardless of the depth when STC correction is performed. That is, an effect of eliminating the influence of attenuation from the luminance value of a B-mode image can be obtained.

On the other hand, when the frequency spectrum of ultrasound is calculated and the analysis result thereof is used as in the present embodiment, it may be difficult to eliminate the influence of attenuation resulting from propagation of ultrasound accurately even when STC correction is performed. This is because although an attenuation amount generally differs depending on a frequency (see Equation (1) below), the amplification factor of STC correction changes depending on a distance only and is not dependent on a frequency.

In order to solve the above-described problem (that is, a problem that when the frequency spectrum of ultrasound is calculated and the analysis result thereof is used as in the present embodiment, it may be difficult to eliminate the influence of attenuation resulting from propagation of ultrasound accurately even when STC correction is performed), a STC-corrected reception signal may be output when a B-mode image is generated, and another transmission different from transmission for generating a B-mode image may be performed to output a non-STC-corrected reception signal when an image based on a frequency spectrum is generated. However, in this case, there is a problem that the frame rate of image data generated based on the reception signal decreases.

Therefore, in the present embodiment, the amplification correction unit 331 corrects an amplification factor of a STC-corrected signal for B-mode images in order to eliminate the influence of STC correction while maintaining the frame rate of generated image data.

The frequency analysis unit 332 samples RF data of respective sound rays which are amplification-corrected by the amplification correction unit 331 at predetermined time intervals to generate sample data. The frequency analysis unit 332 performs FFT processing on a sample data group to calculate a frequency spectrum at a plurality of positions (data positions) on the RF data. The "frequency spectrum" mentioned herein means a "frequency distribution of intensity at a certain reception depth z" obtained by performing FFT processing on a sample data group. Moreover, the "intensity" mentioned herein indicates one of parameters such as, for example, a voltage of an echo signal, an electric power of an echo signal, a sound pressure of an ultrasound echo, or an acoustic energy of an ultrasound echo, an amplitude of these parameters, a time integral value of the parameters, or one of the combinations thereof.

Generally, when an observation target is a living tissue, a frequency spectrum shows different tendencies depending on the characteristics of the living tissue scanned by ultrasound. This is because a frequency spectrum is correlated with the size, the number, the density, the acoustic impedance, or the like of scatters scattering the ultrasound. The examples of the "characteristics of living tissue" mentioned herein include a malignant tumor (cancer), a benign tumor, an endocrine tumor, a mucinous tumor, a normal tissue, a cyst, and a vascular channel.

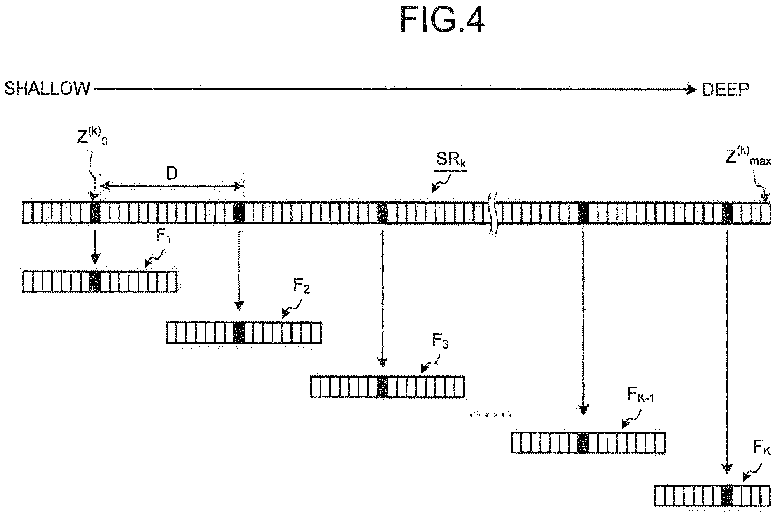

FIG. 4 is a diagram schematically illustrating a data arrangement in one sound ray of an ultrasound signal. In the sound ray $SR_k$ illustrated in the drawing, a white or black rectangle means the data at one sample point. Moreover, in the sound ray $SR_k$, the data located on the right side is sample data from a deep portion measured from the ultrasound transducer 21 along the sound ray SRk (see arrows in FIG. 4). The sound ray $SR_k$ is discretized at a time interval corresponding to a sampling frequency (for example, 50 MHz) of the A/D conversion performed by the transceiving unit 31. Although FIG. 4 illustrates a case where the eighth data position of the sound ray $SR_k$ with the number k is set as the initial value $Z^{(k)}_0$ in the direction of the reception depth z, the position of the initial value may be set arbitrarily. The calculation result obtained by the frequency analysis unit 332 is obtained as a complex number and stored in the storage unit 37.

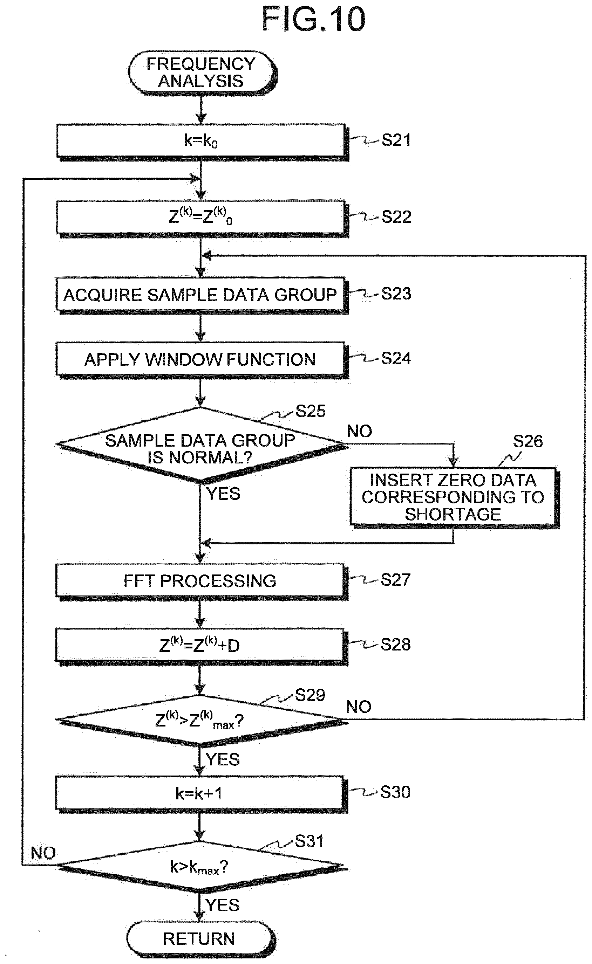

The data group $F_j$ (j = 1, 2, ..., K) illustrated in FIG. 4 is a sample data group to be subjected to the FFT process. In general, in order to perform the FFT process, it is necessary for the sample data group to have data number of power of 2. In this sense, the sample data group $F_j$ (j = 1, 2, ..., K-1) is a normal data group of which the number of pieces of data is 16 (= $2^4$). On the other hand, the sample data group $F_K$ is an abnormal data group of which the number of pieces of data is 12. When the FFT process is performed on an abnormal data group, a process of generating a normal sample data group is performed by inserting zero data corresponding to the shortage. This will be described in detail when describing the process of the frequency analysis unit 332 (see FIG. 10).

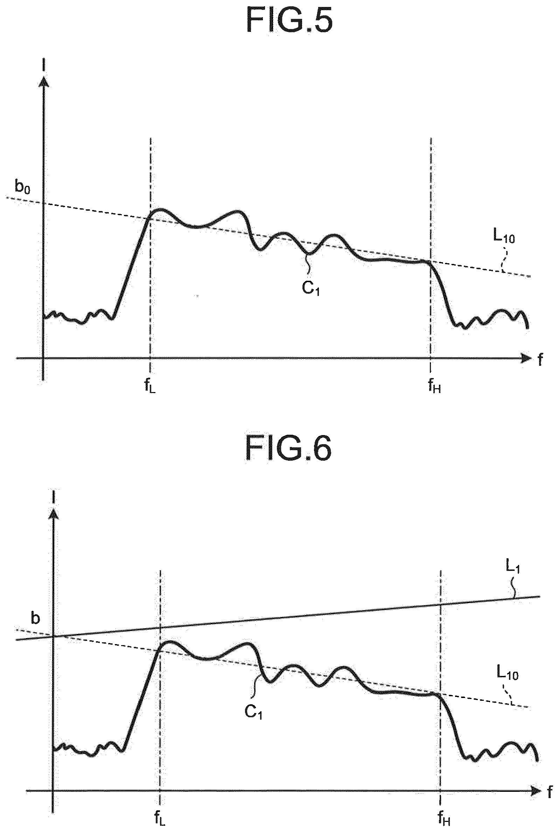

FIG. 5 is a diagram illustrating an example of the frequency spectrum calculated by the frequency analysis unit 332. In FIG. 5, the horizontal axis represents the frequency f. Moreover, in FIG. 5, the vertical axis represents a common logarithm (decibel expression) $I = 10\log_{10}(I_0/I_C)$ of an amount obtained by dividing an intensity $I_0$ by a reference intensity $I_C$ (constant). A straight line $L_{10}$ illustrated in FIG. 5 will be described later. In the embodiment, curves and straight lines are sets of discrete points.

In the frequency spectrum $C_1$ illustrated in FIG. 5, a lower limit frequency $f_L$ and an upper limit frequency $f_H$ of the frequency band used for a subsequent computation operation are parameters determined on the basis of the frequency band of the ultrasound transducer 21, the frequency band of the pulse signal transmitted by the transceiving unit 31, and the like. Hereinafter, in FIG. 5, the frequency band determined by the lower limit frequency $f_L$ and the upper limit frequency $f_H$ is referred to as a "frequency band F".

The feature calculation unit 333 calculates features of a plurality of frequency spectra on the basis of the determination result obtained by the valid region determining unit 334, calculates corrected features of the respective frequency spectra by performing attenuation correction for eliminating the influence of attenuation of ultrasound with respect to features (hereinafter referred to as pre-correction features) of the respective frequency spectra in each of a plurality of attenuation factor candidate values that give different attenuation characteristics when ultrasound propagate through an observation target, and sets an attenuation image optimal for the observation target among a plurality of attenuation factor candidate values using the corrected feature or performs attenuation correction using a predetermined attenuation factor.

The feature calculation unit 333 includes an approximation unit 333a that calculates a feature of a frequency spectrum (hereinafter, referred to as a pre-correction feature) before performing an attenuation correction process by approximating the frequency spectrum with a straight line, an attenuation correction unit 333b that calculates a feature by performing an attenuation correction process with respect to the pre-correction feature calculated by the approximation unit 333a, and an optimal attenuation factor setting unit 333c that sets an optimal attenuation factor among a plurality of attenuation factor candidate values on the basis of a statistical variation in the corrected features calculated by the attenuation correction unit 333b for all frequency spectra.

The approximation unit 333a performs a regression analysis of the frequency spectrum in a predetermined frequency band and approximates the frequency spectrum with a linear equation (regression line) to calculate the pre-correction feature featuring the approximated linear equation. For example, in the case of the frequency spectrum $C_1$ illustrated in FIG. 6, the approximation unit 333a obtains the regression line $L_{10}$ by performing regression analysis in the frequency band F and approximating the frequency spectrum $C_1$ by a linear equation. In other words, the approximation unit 333a calculates, as the pre-correction feature, a mid-band fit $c_0 = a_0 f_M + b_0$ which is a value on the regression line $L_{10}$ having slope $a_0$ of the regression line, intercept $b_0$, and center frequency $f_M = (f_L + f_H)/2$ of the frequency band F.

Among the three pre-correction features, the slope $a_0$ has a correlation with the size of the scatterer of the ultrasound, and it is generally considered that the larger the scatterer, the smaller the slope. The intercept $b_0$ has a correlation with the size of the scatterer, a difference in acoustic impedance, the number density (concentration) of the scatterers, and the like. Specifically, it is considered that the intercept $b_0$ has a larger value as the size of the scatterer is larger, has a larger value as the difference in acoustic impedance is larger, and has a larger value as the number density of the scatterers is larger. The mid-band fit $c_0$ is an indirect parameter derived from the slope $a_0$ and the intercept $b_0$ and gives the intensity of the spectrum at the center within the effective frequency band. For this reason, it is considered that the mid-band fit $c_0$ has a certain degree of correlation with luminance of the B-mode image in addition to the size of the scatterer, a difference in acoustic impedance, and the number density of the scatterers. Moreover, the feature calculation unit 333 may approximate the frequency spectrum with a polynomial of second or higher order by regression analysis.

The correction performed by the attenuation correction unit 333b will be described. In general, the attenuation amount A(f,z) of the ultrasound is an attenuation occurring while the ultrasound reciprocates between the reception depth 0 and the reception depth z, the attenuation amount is defined as a change in intensity before and after the reciprocation (a difference in decibel expression). It is empirically known that the attenuation amount A(f,z) is proportional to the frequency in a uniform tissue and is expressed by Equation (1) below.

$$A(f,z) = 2\alpha zf \qquad (1)$$

Herein, the proportional constant $\alpha$ is an amount called an attenuation factor. Moreover, z is the reception depth of the ultrasound, and f is the frequency. A specific value of the attenuation factor $\alpha$ is determined depending on a portion of a living body when the observation target is the living body. The unit of the attenuation factor $\alpha$ is, for example, dB/cm/MHz. Moreover, in the embodiment, it is possible to change the value of the attenuation factor $\alpha$ by the input from the input unit 35. The attenuation correction unit 333b performs attenuation correction with respect to a predetermined attenuation factor or an attenuation factor candidate value.

The attenuation correction unit 333b performs the attenuation correction on the pre-correction features (slope $a_0$, intercept $b_0$, and mid-band fit $c_0$) extracted by the approximation unit 333a according to Equations (2) to (4) below to calculate features "a", "b", and "c".

$$a = a_0 + 2\alpha z \qquad (2)$$

$$b = b_0 \qquad (3)$$

$$c = c_0 + A(f_M, z) = c_0 + 2\alpha z f_M \ (= af_M + b) \qquad (4)$$

As apparent from Equations (2) and (4), the attenuation correction unit 333b performs the correction such that the larger the reception depth z of ultrasound, the larger becomes the correction amount. Moreover, according to Equation (3), the correction on the intercept is the identity transformation. This is because the intercept is a frequency component corresponding to frequency 0 (Hz) and is not influenced by the attenuation.

FIG. 6 is a diagram illustrating a straight line having the features "a", "b", and "c" calculated by the attenuation correction unit 333b as parameters. The equation of the straight line $L_1$ is expressed as follows.

$$I = af + b = (a_0 + 2\alpha z)f + b_0 \qquad (5)$$

As apparent from Equation (5), the straight line $L_1$ has a larger slope ($a > a_0$) and the same intercept ($b = b_0$) in comparison with the straight line $L_{10}$ before the attenuation correction.

The optimal attenuation factor setting unit 333c sets an attenuation factor candidate value in which a statistical variation in the corrected feature that the attenuation correction unit 333b has calculated for each attenuation factor candidate value with respect to all frequency spectra is smallest as an optimal attenuation factor. In the present embodiment, a variance is used as a quantity indicating the statistical variation. In this case, the optimal attenuation factor setting unit 333c sets an attenuation factor candidate value in which a variance is smallest as an optimal attenuation factor. Two corrected features are independent among the three corrected features a, b, and c. Moreover, the corrected feature b does not depend on an attenuation factor. Therefore, when an operating time is set for the corrected features a and c, the optimal attenuation factor setting unit 333c may calculate a variance of any one of the corrected features a and c.

However, the corrected feature used when the optimal attenuation factor setting unit 333c sets the optimal attenuation factor is preferably the same type as the corrected feature used when a feature image data generation unit 342 generates feature image data. That is, it is preferable that a variance of the corrected feature a is applied when the feature image data generation unit 342 generates feature image data using a slope as a corrected feature and that a variance of the corrected feature c is applied when the feature image data generation unit 342 generates feature image data using a mid-band fit as a corrected feature. This is because Equation (1) that gives an attenuation amount A(f,z) is an ideal equation and practically, Equation (6) below is appropriate.

$$A(f,z) = 2\alpha zf + 2\alpha_1 z \qquad (6)$$

$\alpha_1$ of the second term on the right side of Equation (6) is a coefficient indicating the magnitude of a change in a signal intensity changing in proportion to the reception depth z of ultrasound and is a coefficient indicating the change in a signal intensity occurring due to non-uniformity of an observation target tissue or a change in the number of channels during beam synthesis. Since the second term on the right side of Equation (6) is present, when feature image data is generated using a mid-band fit as a corrected feature, it is possible to correct attenuation accurately by setting the optimal attenuation factor using a variance of the corrected feature c (see Equation (4)). On the other hand, when feature image data is generated using a slope which is a coefficient proportional to frequency f, it is possible to correct attenuation accurately while eliminating the influence of the second terminal on the right side by setting the optimal attenuation factor using a variance of the corrected feature a. For example, the unit of the coefficient $\alpha_1$ is dB/cm when the unit of the attenuation factor $\alpha$ is dB/cm/MHz.

Here, the reason why an optimal attenuation factor can be set on the basis of a statistical variation will be described. It is thought that, when an attenuation factor optimal for an observation target is applied, a feature converges to a value unique to the observation target and a statistical variation decreases regardless of the distance between the observation target and the ultrasound transducer 21. On the other hand, when an attenuation factor candidate value that is not suitable for an observation target is set as an optimal attenuation factor, since attenuation correction is excessive or insufficient, it is thought that a variation occurs in the feature according to the distance between the observation target and the ultrasound transducer 21 and a statistical variation of the feature increases. Therefore, an attenuation factor candidate value in which the statistical variation is smallest can be said to be an optimal attenuation factor of the observation target.

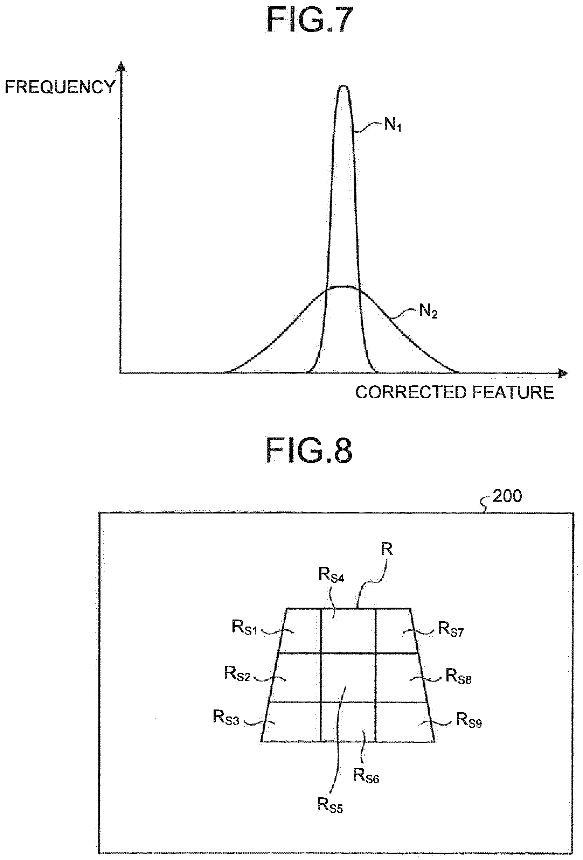

FIG. 7 is a diagram schematically illustrating a distribution example of corrected features attenuated and corrected for the same observation target on the basis of two different attenuation factor candidate values. In FIG. 7, a horizontal axis represents a corrected feature and a vertical axis represents a frequency. The sums of the frequencies of two distribution curves $N_1$ and $N_2$ illustrated in FIG. 7 are equal. In FIG. 7, the distribution curve $N_1$ has a smaller statistical variation (variance) of the feature and has a steeper mountain shape as compared to the current $N_2$. Therefore, when an optimal attenuation factor is set from two attenuation factor candidate values corresponding to the two distribution curves $N_1$ and $N_2$, the optimal attenuation factor setting unit 333c sets an attenuation factor candidate value corresponding to the distribution curve $N_1$ as an optimal attenuation factor.

The valid region determining unit 334 determines whether a target region is a region that does not include a noise region and is a region (a valid region) valid for generating a feature image on the basis of the feature calculated by the feature calculation unit 333. Here, a noise region is a low echo region and is a region that includes water, a cyst, distant noise, or the like. A low echo region contains many noise components and it may be difficult to calculate a feature appropriately.

FIG. 8 is a diagram for describing a region of interest set by the ultrasound observation device 3 and division regions obtained by dividing the region of interest. In the present embodiment, a region of interest R in a B-mode image is set as a region in which a feature is calculated. As illustrated in FIG. 8, in the present embodiment, a plurality of division regions $R_{S1}$ to $R_{S9}$ obtained by dividing a trapezoidal region of interest R by segmenting the same in a vertical direction and a horizontal direction of a display region 200 of a B-mode image.

The valid region determining unit 334 calculates an average value of features calculated by the feature calculation unit 333 for each of division regions (division regions $R_{S1}$ to $R_{S9}$) and compares the average value with a predetermined threshold to thereby determine whether a determination target region is a valid region. Specifically, the valid region determining unit 334 determines that the division region is a valid region when an average value of the corrected features c in the determination target division regions among the corrected features c (mid-band fit) calculated by the attenuation correction unit 333b is equal to or larger than a threshold and determines that the division region is not a valid region (is a non-valid region) when the average value is smaller than the threshold. The threshold mentioned herein is a value set on the basis of a value of a mid-band fit calculated from an echo signal of a low echo region when a valid region or a non-valid region is determined using a mid-band fit, for example, as described above. The valid region determining unit 334 functions as a comparison unit.

The image processing unit 34 is configured to include a B-mode image data generation unit 341 that generates B-mode image data which is an ultrasound image to be displayed by converting the amplitude of an echo signal to a luminance and a feature image data generation unit 342 that generates feature image data in which the feature calculated by the feature calculation unit 333 is displayed together with the B-mode image in correlation with visual information.

The B-mode image data generation unit 341 generates the B-mode image data by performing signal processing using known techniques such as gain processing and contrast processing on the B-mode reception data received from the signal processing unit 32 and performing data thinning according to a data step width determined according to the display range of images on the display device 4. The B-mode image is a grayscale image in which the values of R (red), G (green), and B (blue) which are variables when an RGB color system is used as a color space are matched.

The B-mode image data generation unit 341 performs coordinate transformation for rearranging the B-mode reception data from the signal processing unit 32 so that the scanning range can be spatially correctly expressed and, after that, performs interpolation between the B-mode reception data to fill gaps between the B-mode reception data and generate the B-mode image data. The B-mode image data generation unit 341 outputs the generated B-mode image data to the feature image data generation unit 342.

The feature image data generation unit 342 generates feature image data by superimposing visual information related to the features calculated by the feature calculation unit 333 on each pixel of the image in the B-mode image data. The feature image data generation unit 342 allocates, for example, to a pixel region corresponding to the data amount of one sample data group $F_j$ (j = 1, 2, ..., K) illustrated in FIG. 4, visual information corresponding to the feature of the frequency spectrum calculated from the sample data group $F_j$. For example, the feature image data generation unit 342 generates feature image data by associating the hue as the visual information with any one of the above-described slope, intercept, and mid-band fit. The feature image data generation unit 342 may generate the feature image data by correlating hue with one of two features selected from a slope, an intercept, and a mid-band fit and correlating brightness with the other. Examples of the visual information related to the feature include variables of a color space constituting a predetermined color system such as hue, saturation, brightness, luminance value, R (red), G (green), and B (blue).

Here, the feature image data generated by the feature image data generation unit 342 is such image data that a feature image of a region corresponding to a region of interest (ROI) segmented by a specific depth width and a sound ray width in a scanning region S is displayed on the display device 4.

The control unit 36 is realized using a general purpose processor such as a CPU having computation and control functions or specific purpose integrated circuit such as an ASIC or an FPGA. The control unit 36 reads the information stored and retained by the storage unit 37 from the storage unit 37 and executes various computation processes related to a method of operating the ultrasound observation device 3, so as to control the ultrasound observation device 3 in a unified manner. It is also possible to configure the control unit 36 using a general purpose processor common to the signal processing unit 32 and the computing unit 33 or a specific purpose integrated circuit.

The storage unit 37 stores the plurality of features calculated for each frequency spectrum by the feature calculation unit 333 and the image data generated by the image processing unit 34. Moreover, the storage unit 37 includes a feature information storage unit 371 that stores a plurality of features calculated for each frequency spectrum according to an attenuation factor candidate value by the attenuation correction unit 333b and a variance that gives a statistical variation of the plurality of features in correlation with the attenuation factor candidate value, a determination information storage unit 372 that stores a threshold for allowing the valid region determining unit 334 to determine whether a determination target region is a valid region, and an attenuation factor information storage unit 373 that stores an attenuation factor for calculating a corrected feature before the determination of the valid region determining unit 334 and an attenuation factor for performing attenuation correction on the feature of a region which is determined to be a non-valid region by the valid region determining unit 334.

In addition to the above-mentioned information, the storage unit 37 stores, for example, information necessary for the amplification process (a relationship between the amplification factor and the reception depth illustrated in FIG. 2), information necessary for the amplification correction process (a relationship between the amplification factor and the reception depth illustrated in FIG. 3), information necessary for the attenuation correction process (see Equation (1)), information related to a window function (Hamming, Hanning, Blackman, or the like) necessary for a frequency analysis process, and the like. Moreover, the storage unit 37 stores a corrected feature which is the corrected feature calculated by the attenuation correction unit 333b and which the valid region determining unit 334 uses for determination.

Moreover, the storage unit 37 stores various programs including an operation program for executing the method of operating the ultrasound observation device 3. The operation program may also be recorded on a computer-readable recording medium such as a hard disk, a flash memory, a CD-ROM, a DVD-ROM, or a flexible disk and distributed widely. Moreover, the above-described various programs may also be acquired by downloading via a communication network. The communication network mentioned herein is realized by, for example, an existing public line network, a local area network (LAN), a wide area network (WAN), and the like and may be wired or wireless.

The storage unit 37 having the above configuration is realized using a read only memory (ROM) in which various programs and the like are preliminarily installed, a random access memory (RAM) for storing computation parameters and data of each process, and the like.

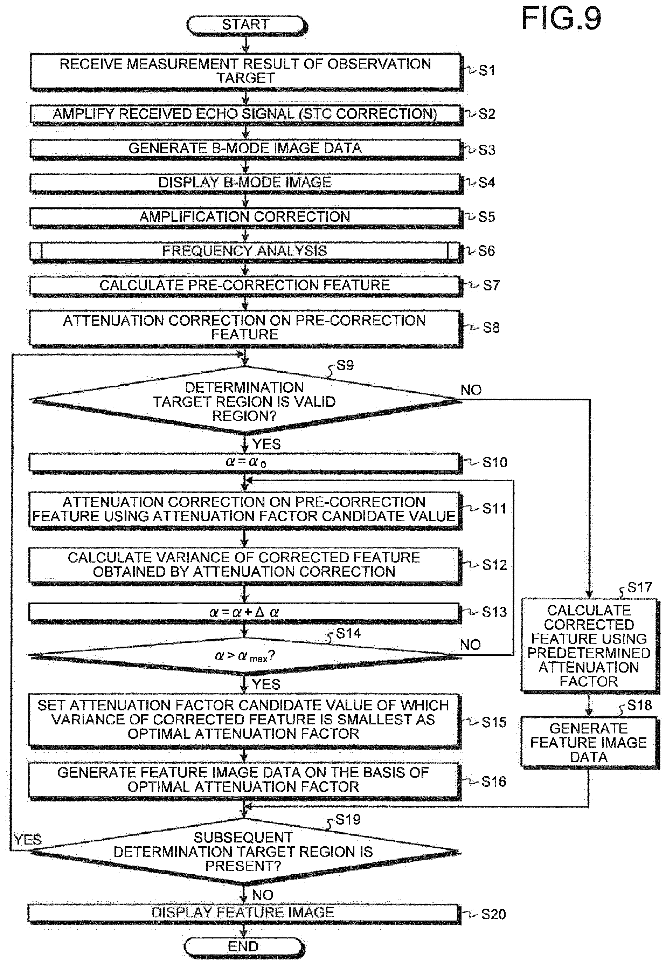

FIG. 9 is a flowchart illustrating the overview of the processes performed by the ultrasound observation device 3 having the above-described configuration. First, the ultrasound observation device 3 receives an echo signal as a measurement result of an observation target by the ultrasound transducer 21 from the ultrasound endoscope 2 (Step S1).

Upon receiving the echo signal from the ultrasound transducer 21, the signal amplification unit 311 amplifies the echo signal (Step S2). Here, for example, the signal amplification unit 311 performs amplification (STC correction) of the echo signal on the basis of the relationship between the amplification factor and the reception depth illustrated in FIG. 2, for example.

Subsequently, the B-mode image data generation unit 341 generates a B-mode image data using the echo signal amplified by the signal amplification unit 311 and outputs the B-mode image data to the display device 4 (Step S3). Upon receiving the B-mode image data, the display device 4 displays a B-mode image corresponding to the B-mode image data (Step S4).

The amplification correction unit 331 performs amplification correction on the signal output from the transceiving unit 31 so that the amplification factor is constant regardless of the reception depth (Step S5). Herein, for example, the amplification correction unit 331 performs amplification correction such that the relationship between the amplification factor and the reception depth illustrated in FIG. 3, for example, is established.

After that, the frequency analysis unit 332 calculates frequency spectra for all the sample data groups by performing frequency analysis using the FFT process (Step S6: frequency analysis step). FIG. 10 is a flowchart illustrating an overview of the process executed by the frequency analysis unit 332 in Step S6. Hereinafter, the frequency analysis process will be described in detail with reference to the flowchart illustrated in FIG. 10.

First, the frequency analysis unit 332 sets a counter k for identifying an analysis target sound ray as $k_0$ (Step S21).

Subsequently, the frequency analysis unit 332 sets an initial value $Z^{(k)}{}_0$ of a data position (corresponding to a reception depth) $Z^{(k)}$ representing a series of data groups (sample data groups) acquired for the FFT process (Step S22). For example, FIG. 4 illustrates a case where the eighth data position of the sound ray $SR_k$ is set as the initial value $Z^{(k)}{}_0$ as described above.

After that, the frequency analysis unit 332 acquires the sample data group (Step S23), and applies a window function stored in the storage unit 37 to the acquired sample data group (Step S24). In this manner, by applying the window function to the sample data group, it is possible to prevent the sample data group from becoming discontinuous at the boundary and to prevent artifacts from occurring.

Subsequently, the frequency analysis unit 332 determines whether the sample data group at the data position $Z^{(k)}$ is a normal data group (Step S25). As described with reference to FIG. 4, the sample data group needs to have a number of pieces of data of a power of 2. Hereinafter, the number of pieces of data of the normal sample data group is $2^n$ (n is a positive integer). In the present embodiment, the data position $Z^{(k)}$ is set to be the center of the sample data group to which $Z^{(k)}$ belongs as much as possible. Specifically, since the number of pieces of data of the sample data group is $2^n$, $Z^{(k)}$ is set to the $2^n/2(= 2^{n-1})$-th position close to the center of the sample data group. In this case, the fact that the sample data group is normal denotes that there are $2^{n-1}-1$ (= N) pieces of data before the data position $Z^{(k)}$ and there are $2^{n-1}$ (= M) pieces of data after the data position $Z^{(k)}$. In the case of FIG. 4, the sample data groups $F_1$, $F_2$, $F_3$, ..., and $F_{K-1}$ are normal. Moreover, in FIG. 4, the case of n=4 (N = 7, M = 8) is illustrated.

As a result of the determination in Step S25, in a case where the sample data group at the data position $Z^{(k)}$ is normal (Step S25: Yes), the frequency analysis unit 332 proceeds to Step S27 to be described later.

As a result of the determination in Step S25, in a case where the sample data group at the data position $Z^{(k)}$ is not normal (Step S25: No), the frequency analysis unit 332 generates a normal sample data group by inserting zero data corresponding to the shortage (Step S26). In the sample data group (for example, the sample data group $F_K$ in FIG. 4) that is determined not to be normal in Step S25, the window function is applied before adding the zero data. For this reason, no data discontinuity occurs even if the zero data is inserted into the sample data group. After Step S26, the frequency analysis unit 332 proceeds to Step S27 to be described later.

In Step S27, the frequency analysis unit 332 performs the FFT process using the sample data group to obtain a frequency spectrum which is the frequency distribution of amplitude (Step S27).

Subsequently, the frequency analysis unit 332 changes the data position $Z^{(k)}$ by the step width D (Step S28). It is assumed that the storage unit 37 previously stores the step width D. In FIG. 4, the case of D = 15 is illustrated. It is desirable that the step width D is allowed to coincide with the data step width used by the B-mode image data generation unit 341 at the time of generating the B-mode image data. However, in a case where it is desired to reduce the computation amount in the frequency analysis unit 332, a value larger than the data step width may be set as the width D.

After that, the frequency analysis unit 332 determines whether or not the data position $Z^{(k)}$ is larger than the maximum value $Z^{(k)}{}_{max}$ on the sound ray $SR_k$ (Step S29). In a case where the data position $Z^{(k)}$ is larger than the maximum value $Z^{(k)}{}_{max}$ (Step S29: Yes), the frequency analysis unit 332 increments the counter k by 1 (Step S30). This means that the process is shifted to an adjacent sound ray. On the other hand, in a case where the data position $Z^{(k)}$ is equal to or smaller than the maximum value $Z^{(k)}{}_{max}$ (Step S29: No), the frequency analysis unit 332 returns to Step S23. In this manner, the frequency analysis unit 332 performs the FFT process on $[(Z^{(k)}{}_{max} - Z^{(k)}{}_0 + 1/D + 1]$ sample data groups on the sound ray $SR_k$. Here, [X] represents the largest integer not exceeding X.

After Step S30, the frequency analysis unit 332 determines whether or not the counter k is larger than the maximum value $k_{max}$, (Step S31). In a case where the counter k is larger than the maximum value $k_{max}$ (Step S31: Yes), the frequency analysis unit 332 ends a series of the frequency analysis processes. On the other hand, in a case where the counter k is equal to or smaller than the maximum value $k_{max}$ (Step S31: No), the frequency analysis unit 332 returns to Step S22. The maximum value $k_{max}$ is set to a value arbitrarily entered by the user such as a doctor through the input unit 35 or set in advance in the storage unit 37.

In this manner, the frequency analysis unit 332 performs the FFT process multiple times for each of ($k_{max}$ - $k_0$ + 1) sound rays within the analysis target region. The result of the FFT process is stored in the feature information storage unit 371 together with the reception depth and the reception direction.

Following the frequency analysis process in Step S6 described above, the feature calculation unit 333 calculates the pre-correction features of the plurality of frequency spectra, performs the attenuation correction for eliminating the influence of the attenuation of ultrasound on the pre-correction feature of each frequency spectrum in each of a plurality of attenuation factor candidate values that give different attenuation characteristics when ultrasound propagates through an observation target to calculate the corrected feature of each frequency spectrum, and sets an attenuation factor optimal for the observation target among a plurality of attenuation factor candidate values using the corrected feature (Steps S7, S8, and S10 to S18: feature calculation step). Hereinafter, the processes of Steps S7 to S18 will be described in detail.

In Step S7, the approximation unit 333a performs the regression analysis on each of the frequency spectra generated by the frequency analysis unit 332 to calculate the pre-correction feature corresponding to each frequency spectrum (Step S7). Specifically, the approximation unit 333a approximates each frequency spectrum with a linear equation by performing the regression analysis and calculates the slope $a_0$, the intercept $b_0$, and the mid-band fit $c_0$ as pre-correction features. For example, the straight line $L_{10}$ illustrated in FIG. 5 is a regression line approximated by the approximation unit 333a to the frequency spectrum $C_1$ of the frequency band F by performing the regression analysis.

Subsequently, the attenuation correction unit 333b calculates the corrected feature by performing the attenuation correction using the predetermined attenuation factor stored in the attenuation factor information storage unit 373 on the pre-correction feature approximated to each frequency spectrum by the approximation unit 333a (Step S8). The straight line $L_1$ illustrated in FIG. 6 is an example of a straight line obtained by the attenuation correction unit 333b performing the attenuation correction process.

When the corrected feature is calculated in Step S8, the valid region determining unit 334 determines whether a determination target division region is a valid region or a non-valid region using the corrected feature (Step S9: comparing step). In the present embodiment, the corrected feature c (mid-band fit) is used, and it is determined that the division region is a valid region if the average value of the corrected feature c is equal to or larger than the threshold and determines that the division region is a non-valid region if the average value is smaller than the threshold by referring to the determination information storage unit 372. Here, when the valid region determining unit 334 determines that the determination target division region is a valid region (Step S9: Yes), the control unit 36 proceeds to Step S10. On the other hand, when the valid region determining unit 334 determines that the determination target division region is a non-valid region (Step S9: No), the control unit 36 proceeds to Step S17.

In Step S10, the optimal attenuation factor setting unit 333c sets the value of the attenuation factor candidate value $\alpha$ to be applied when performing attenuation correction to be described later to a predetermined initial value $\alpha_0$ (Step S10). The value of the initial value $\alpha_0$ may be stored in advance in the storage unit 37 so that the optimal attenuation factor setting unit 333c refers to the storage unit 37.

Subsequently, the attenuation correction unit 333b performs attenuation correction using the attenuation factor candidate value $\alpha$ with respect to the pre-correction feature that the approximation unit 333a has approximated for each frequency spectrum to calculate the corrected feature and stores the corrected feature in the feature information storage unit 371 together with the attenuation factor candidate value $\alpha$ (Step S11).

In Step S11, the attenuation correction unit 333b calculates the corrected feature by inserting the data position $Z = (f_{sp}/2v_s)Dn$ obtained using the data array of the sound rays of ultrasound signals to the reception depth z in Equations (2) and (4) described above. Here, $f_{sp}$ is the data sampling frequency, $v_s$ is the sound velocity, D is the data step width, and n is the number of data steps from the first data of the sound ray to the data position of the sample data group to be processed. For example, if the data sampling frequency $f_{sp}$ is 50 MHz, the sound velocity $v_s$ is 1530 m/sec, and the data arrangement illustrated in FIG. 4 is adopted so that the step width D is 15, $Z = 0.2295n$ (mm).

The optimal attenuation factor setting unit 333c calculates a variance of a representative corrected feature among a plurality of corrected features obtained by the attenuation correction unit 333b performing attenuation correction on each frequency spectrum and stores the variance in the feature information storage unit 371 in correlation with the attenuation factor candidate value $\alpha$ (Step S12). When the corrected feature is the slope a and the mid-band fit c, the optimal attenuation factor setting unit 333c calculates a variance of the corrected feature c, for example. In Step S13, it is preferable that the optimal attenuation factor setting unit 333c applies a variance of the corrected feature a when the feature image data generation unit 342 generates feature image data using a slope and applies a variance of the corrected feature c when the feature image data generation unit 342 generates feature image data using a mid-band fit.

After that, the optimal attenuation factor setting unit 333c increases the value of the attenuation factor candidate value $\alpha$ by $\Delta\alpha$ (Step S13) and compares the attenuation factor candidate value $\alpha$ after the increase with a predetermined maximum value $\alpha_{max}$ (Step S14). When the comparison result in Step S14 shows that the attenuation factor candidate value $\alpha$ is larger than the maximum value $\alpha_{max}$ (Step S14: Yes), the ultrasound observation device 3 proceeds to Step S15. On the other hand, when comparison result in Step S14 shows that the attenuation factor candidate value $\alpha$ is equal to or smaller than the maximum value $\alpha_{max}$ (Step S14: No), the ultrasound observation device 3 returns to Step S11.

In Step S15, the optimal attenuation factor setting unit 333c sets an attenuation factor candidate value of which the variance is the smallest as an operating time by referring to the variances of respective attenuation factor candidate values stored in the feature information storage unit 371 (Step S15).

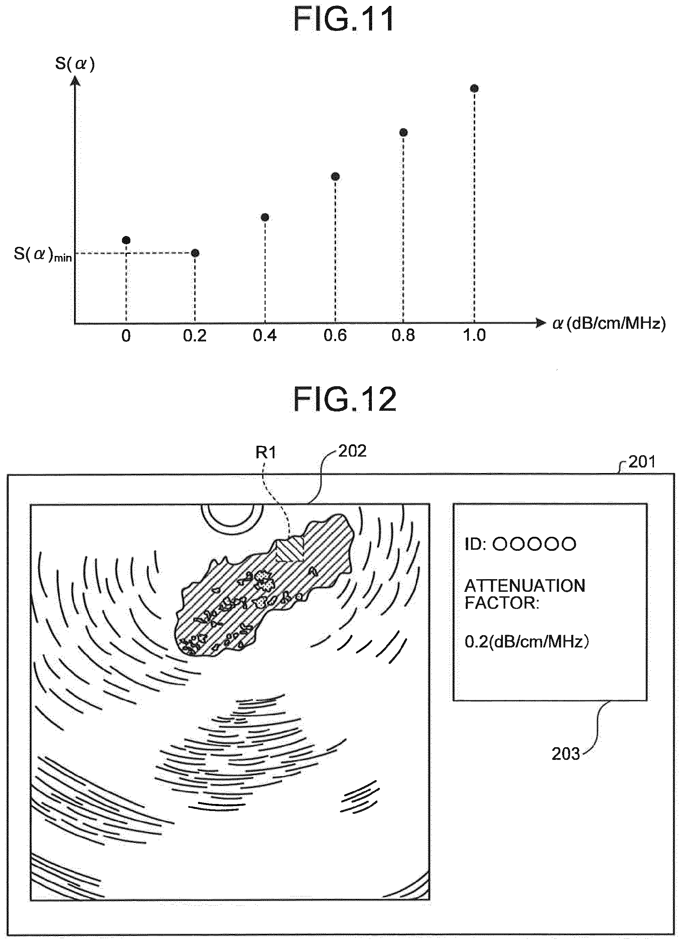

FIG. 11 is a diagram illustrating an overview of the process performed by the optimal attenuation factor setting unit 333c. FIG. 11 is a diagram illustrating an example of a relationship between the attenuation factor candidate value $\alpha$ and the variance $S(\alpha)$ when $\alpha_0 = 0$ (dB/cm/MHz), $\alpha_{max} = 1.0$ (dB/cm/MHz), $\Delta\alpha = 0.2$ (dB/cm/MHz). In the case of FIG. 11, the variance amounts to its minimum value $S(\alpha)_{min}$ when the attenuation factor candidate value $\alpha$ is 0.2 (dB/cm/MHz). Therefore, in the case of FIG. 11, the optimal attenuation factor setting unit 333c sets $\alpha = 0.2$ (dB/cm/MHz) as the optimal attenuation factor.

The approximation unit 333a may calculate a curve that interpolates the value of a variance $S(\alpha)$ at the attenuation factor candidate value $\alpha$ by performing regression analysis before the optimal attenuation factor setting unit 333c sets the optimal attenuation factor. After that, the minimum value $S(\alpha)'_{min}$ in a range of 0 (dB/cm/MHz) $\leq \alpha \leq 1.0$ (dB/cm/MHz) may be calculated for this curve, and the value $\alpha'$ of the attenuation factor candidate value at that time may be set as the optimal attenuation factor. In the case of FIG. 11, the optimal attenuation factor $\alpha'$ is a value between 0 (dB/cm/MHz) and 0.2 (dB/cm/MHz).

For each pixel in the B-mode image data generated by the B-mode image data generation unit 341, the feature image data generation unit 342 generates feature image data by superimposing the visual information (for example, hue) correlated with the corrected feature based on the optimal attenuation factor set in Step S15 on the position corresponding to the determination target division region and adding the information on the optimal attenuation factor thereto (Step S16: feature image data generation step).

In Step S17, the attenuation correction unit 333b calculates a corrected feature by performing attenuation correction on the pre-correction feature that the approximation unit 333a has approximated to each frequency spectrum by referring to the attenuation factor information storage unit 373 and stores the calculated corrected feature in the feature information storage unit 371 (Step S17). Here, the attenuation factor in the non-valid region is set to an arbitrary value in the range of 0.0 to 2.0 (dB/cm/MHz).

For each pixel in the B-mode image data generated by the B-mode image data generation unit 341, the feature image data generation unit 342 generates feature image data by superimposing the visual information (for example, hue) correlated with the corrected feature calculated in Step S17 on the position corresponding to the determination target division region and adding the information on the attenuation factor thereto (Step S18: feature image data generation step).

In Step S19, the control unit 36 determines whether a subsequent determination target division region is present (Step S19). Here, the control unit 36 proceeds to Step S20 when it is determined that the subsequent determination target division region is not present (Step S19: No). On the other hand, the control unit 36 returns to Step S9 when it is determined that the subsequent determination target division region is present (Step S19: Yes).

After that, in Step S20, under the control of the control unit 36, the display device 4 displays a feature image corresponding to the feature image data generated by the feature image data generation unit 342 (Step S20). FIG. 12 is a diagram schematically illustrating a display example of a feature image on the display device 4. A feature image 201 illustrated in the drawing has a superimposed image display portion 202 for displaying an image in which visual information on a feature is superimposed on a B-mode image and an information display portion 203 for displaying identification information or the like of the observation target. Here, a region R1 in the superimposed image display portion 202 corresponds to a region which is determined to be a non-valid region by the valid region determining unit 334. The information display portion 203 may further display information on a feature, information on an approximate equation, image information such as gain and contrast, a determination result obtained by the valid region determining unit 334, and the like. Moreover, the B-mode image corresponding to the feature image may be displayed side by side with the feature image. Moreover, the attenuation factor being displayed may be the attenuation factor for each division region and may be an average value of the attenuation factors of the division regions or an average value of the attenuation factors (optimal attenuation factors) of division regions which are determined to be a valid region.

In the above-described series of processes (Steps S1 to S20), the process of Step S3 and the processes of Steps S5 to S19 may be performed in parallel.

According to an embodiment of the present invention described above, the valid region determining unit 334 determines whether each division region of the region of interest R is a valid region or a non-valid region that includes a noise region on the basis of the corrected feature, and the feature calculation unit 333 calculates the corrected feature on the basis of the optimal attenuation factor or calculates the corrected feature using a predetermined attenuation factor according to the determination result. Therefore, it is possible to calculate the feature appropriately even when a noise region is included.

According to the present embodiment, an attenuation factor optimal for an observation target is set among a plurality of attenuation factor candidate values that give different attenuation characteristics when ultrasound propagates through the observation target and the feature of each of the plurality of frequency spectra is calculated by performing attenuation correction using the optimal attenuation factor. Therefore, it is possible to obtain attenuation characteristics of the ultrasound suitable for the observation target with simple computation and to perform observation using the attenuation characteristics.

According to the present embodiment, since the optimal attenuation factor is set on the basis of the statistical variation of the corrected feature obtained by performing attenuation correction on each frequency spectrum, it is possible to reduce the amount of computation as compare to a conventional technique that performs fitting with a plurality of attenuation models.

In the present embodiment, although the valid region determining unit 334 determines whether each division region is a valid region or a non-valid region using the average value of the corrected features c related to the frequency feature as a physical quantity, the physical quantity is not limited thereto. The physical quantity may be a largest value, a smallest value, or a most frequent value without being limited to the average value. In the embodiment described above, although the corrected feature c is used as the physical quantity, examples of the other physical quantities include the corrected feature a related to the frequency feature, the luminance of a B-mode image that is not related to the frequency feature, a spectrum intensity, a value correlated with the spectrum intensity, a change in an elastography, a sound velocity, and the like. The physical quantity is preferably related to a feature used when generating feature image data. When the physical quantity is not related to the frequency feature, the valid region determining unit 334 may determine whether the region of interest is a valid region on the basis of the physical quantity before the feature calculation unit 333 calculates the corrected feature.

In the present embodiment, for example, the optimal attenuation factor setting unit 333c may calculate optimal attenuation factor corresponding values corresponding to optimal attenuation factors in all frames of an ultrasound image and may set an average value, a median value, or a most frequent value of a predetermined number of optimal attenuation factor corresponding values including the optimal attenuation factor corresponding value in a latest frame. In this case, a change in the optimal attenuation factor is smaller than in the case of setting the optimal attenuation factor in each frame, and the value thereof can be stabilized.

In the present embodiment, the optimal attenuation factor setting unit 333c may set the optimal attenuation factor at a predetermined frame interval of the ultrasound image. In this way, it is possible to reduce the amount of computation dramatically. In this case, when the next optimal attenuation factor is set, the value of the optimal attenuation factor set lastly may be used.

In the present embodiment, although division regions obtained by dividing the trapezoidal region of interest R in a lattice form are set, a region of interest and/or a division region formed by a straight line or a curve extending along the same depth and a straight line extending in a depth direction may be used, and a segment set in a sound ray may be used as a division region.

In the present embodiment, the input unit 35 may be configured to receive the input of a change in the setting of the initial value $\alpha_0$ of the attenuation factor candidate value.

In the present embodiment, a standard deviation, a difference between the largest value and the smallest value of features in a population, or a half-value width of a feature distribution may be used as an example of the quantity that gives a statistical variation. A reciprocal of a variance may be used as the quantity that gives a statistical variation. In this case, an attenuation factor candidate value of which the reciprocal of the variance is the largest is naturally the optimal attenuation factor.

In the present embodiment, the optimal attenuation factor setting unit 333c may calculate the statistical variations of a plurality of types of corrected features and set an attenuation factor candidate value of which the statistical variation is the smallest as the optimal attenuation factor.

In the present embodiment, the attenuation correction unit 333b may calculate the corrected feature by performing attenuation correction on the frequency spectrum using a plurality of attenuation factor candidate values and allowing the approximation unit 333a to perform regression analysis on each frequency spectrum after the attenuation correction.

In the present embodiment, although the feature is calculated for the region of interest only, the feature may be calculated without designating a particular region.

As described above, the present invention may include various embodiments within the scope without departing from the technical idea described in the claims.

Industrial Applicability

As described above, the ultrasound observation device, the method of operating the ultrasound observation device, and the program for operating the ultrasound observation device according to the present invention are useful for calculating the feature appropriately even when a noise region is included.

Reference Signs List

1          ULTRASOUND OBSERVATION SYSTEM

| | |
|---|---|
| 2 | ULTRASOUND ENDOSCOPE |
| 3 | ULTRASOUND OBSERVATION DEVICE |
| 4 | DISPLAY DEVICE |
| 21 | ULTRASOUND TRANSDUCER |
| 31 | TRANSCEIVING UNIT |
| 32 | SIGNAL PROCESSING UNIT |
| 33 | COMPUTING UNIT |
| 34 | IMAGE PROCESSING UNIT |
| 35 | INPUT UNIT |
| 36 | CONTROL UNIT |
| 37 | STORAGE UNIT |
| 201 | FEATURE IMAGE |
| 202 | SUPERIMPOSED IMAGE DISPLAY PORTION |
| 203 | INFORMATION DISPLAY PORTION |
| 311 | SIGNAL AMPLIFICATION UNIT |
| 331 | AMPLIFICATION CORRECTION UNIT |
| 332 | FREQUENCY ANALYSIS UNIT |
| 333 | FEATURE CALCULATION UNIT |
| 333a | APPROXIMATION UNIT |
| 333b | ATTENUATION CORRECTION UNIT |
| 333c | OPTIMAL ATTENUATION FACTOR SETTING UNIT |
| 334 | VALID REGION DETERMINING UNIT |
| 341 | B-MODE IMAGE DATA GENERATION UNIT |
| 342 | FEATURE IMAGE DATA GENERATION UNIT |
| 371 | FEATURE INFORMATION STORAGE UNIT |
| 372 | DETERMINATION INFORMATION STORAGE UNIT |
| 373 | ATTENUATION FACTOR INFORMATION STORAGE UNIT |
| $C_1$ | FREQUENCY SPECTRUM |

**Claims**

1. An ultrasound observation device configured to generate an ultrasound image based on an ultrasound signal acquired by an ultrasound probe including an ultrasound transducer that transmits ultrasound to an observation target and receives ultrasound reflected from the observation target, the ultrasound observation device comprising:

   a frequency analysis unit configured to analyze a frequency of a signal generated based on the ultrasound signal to calculate a plurality of frequency spectra;
   a comparison unit configured to compare a physical quantity based on the ultrasound reflected from the observation target with a threshold set according to the physical quantity; and
   a feature calculation unit configured to calculate a frequency feature based on the frequency spectrum calculated by the frequency analysis unit and a comparison result obtained by the comparison unit.

2. The ultrasound observation device according to claim 1, wherein
   the comparison unit is configured to compare the physical quantity with the threshold for each of a plurality of regions set in the ultrasound image, and
   the feature calculation unit is configured to set an attenuation factor according to the comparison result obtained by the comparison unit to calculate the frequency feature for each of the regions.

3. The ultrasound observation device according to claim 1, wherein
   the physical quantity is one selected from the group consisting of the frequency feature, a luminance of the ultrasound image, a change in an elastography, and a sound velocity.

4. The ultrasound observation device according to claim 3, wherein
   the physical quantity is related to the frequency feature.

5. The ultrasound observation device according to claim 3, wherein
   the physical quantity is a mid-band fit which is a value of a linear equation in a mid-frequency of a predetermined frequency band in the frequency spectrum, the mid-band fit being calculated by the feature calculation unit, the frequency band being approximated to the linear equation.

6. The ultrasound observation device according to claim 1, wherein
the comparison unit is configured to determine whether a low echo region is included based on the physical quantity and the threshold, and
the feature calculation unit is configured to calculate a frequency feature using a predetermined attenuation factor when a determination result obtained by the comparison unit indicates that the low echo region is included.

7. The ultrasound observation device according to claim 6, wherein
the frequency analysis unit is configured to calculate a plurality of frequency spectra, and
when the determination result obtained by the comparison unit indicates that the low echo region is not included, the feature calculation unit is configured to:

> calculate features of the plurality of frequency spectra;
> calculate a corrected feature of each of the frequency spectra by performing attenuation correction for eliminating an influence of attenuation of the ultrasound with respect to the feature of each of the frequency spectra for each of a plurality of attenuation factor candidate values that give different attenuation characteristics when the ultrasound propagates through the observation target; and
> set an optimal attenuation factor optimal for the observation target among the plurality of attenuation factor candidate values using the corrected feature.

8. The ultrasound observation device according to claim 7, wherein
the feature calculation unit is configured to:

> calculate the feature by approximating each of the frequency spectra to an n-th order equation (n is a positive integer);
> calculate a statistical variation of the corrected feature for each of the attenuation factor candidate values; and
> set an attenuation factor candidate value of which the statistical variation is the smallest as the optimal attenuation factor.

9. The ultrasound observation device according to claim 8, wherein
the feature calculation unit is configured to:

> approximate a predetermined frequency band in the frequency spectrum to a linear equation;
> calculate one or a plurality of features among an intercept and a slope of the linear equation and a mid-band fit which is a value of the linear equation at a mid-frequency of the frequency band, the one or plurality of features including any one of the slope and the mid-band fit; and
> set the optimal attenuation factor based on any one of the slope and the mid-band fit.

10. The ultrasound observation device according to claim 9, wherein
the feature calculation unit is configured to:

> set the optimal attenuation factor based on the slope when the slope is calculated as the feature; and
> set the optimal attenuation factor based on the mid-band fit when the mid-band fit is calculated as the feature.

11. The ultrasound observation device according to claim 8, wherein
the feature calculation unit is configured to:

> obtain the statistical variation as a function of the attenuation factor candidate value; and
> set an attenuation factor candidate value for which the statistical variation in the function is the smallest as the optimal attenuation factor.

12. The ultrasound observation device according to claim 1, further comprising: a feature image data generation unit configured to generate feature image data for displaying the frequency feature calculated by the feature calculation unit together with the ultrasound image in correlation with visual information.

13. A method of operating an ultrasound observation device configured to generate an ultrasound image based on an ultrasound signal acquired by an ultrasound probe including an ultrasound transducer that transmits ultrasound to an observation target and receives ultrasound reflected from the observation target, the method comprising:

a frequency analysis step of causing a frequency analysis unit to analyze a frequency of a signal generated based on the ultrasound signal to calculate a plurality of frequency spectra;
a comparison step of causing a comparison unit to compare a physical quantity based on the ultrasound reflected from the observation target with a threshold set according to the physical quantity; and
a feature calculation step of causing a feature calculation unit to calculate a frequency feature based on the frequency spectrum calculated by the frequency analysis unit and a comparison result obtained by the comparison unit.

14. A program for operating an ultrasound observation device configured to generate an ultrasound image based on an ultrasound signal acquired by an ultrasound probe including an ultrasound transducer that transmits ultrasound to an observation target and receives ultrasound reflected from the observation target, the program causing the ultrasound observation device to execute:

a frequency analysis process of causing a frequency analysis unit to analyze a frequency of a signal generated based on the ultrasound signal to calculate a plurality of frequency spectra;
a comparison process of causing a comparison unit to compare a physical quantity based on the ultrasound reflected from the observation target with a threshold set according to the physical quantity; and
a feature calculation process of causing a feature calculation unit to calculate a frequency feature based on the frequency spectrum calculated by the frequency analysis unit and a comparison result obtained by the comparison unit.

FIG.1

DISPLAY DEVICE — 4

ULTRASOUND ENDOSCOPE — 2
ULTRASOUND TRANSDUCER — 21

ULTRASOUND OBSERVATION DEVICE — 1, 3

TRANSCEIVING UNIT — 31
SIGNAL AMPLIFICATION UNIT — 311

SIGNAL PROCESSING UNIT — 32

INPUT UNIT — 35

STORAGE UNIT — 37
FEATURE INFORMATION STORAGE UNIT — 371
DETERMINATION INFORMATION STORAGE UNIT — 372
ATTENUATION FACTOR INFORMATION STORAGE UNIT — 373

CONTROL UNIT — 36

COMPUTING UNIT — 33
AMPLIFICATION CORRECTION UNIT — 331
FREQUENCY ANALYSIS UNIT — 332
FEATURE CALCULATION UNIT — 333
APPROXIMATION UNIT — 333a
ATTENUATION CORRECTION UNIT — 333b
OPTIMAL ATTENUATION FACTOR SETTING UNIT — 333c
VALID REGION DETERMINING UNIT — 334

IMAGE PROCESSING UNIT — 34
B-MODE IMAGE DATA GENERATION UNIT — 341
FEATURE IMAGE DATA GENERATION UNIT — 342

18

# FIG.2

# FIG.3

# FIG.4

SHALLOW ────────────────────────────────────────────────→ DEEP

$Z^{(k)}_0$    $D$    $\underline{SR_k}$    $Z^{(k)}_{max}$

$F_1$

$F_2$

$F_3$

. . . . . .    $F_{K-1}$

$F_K$

EP 3 384 854 A1

# FIG.5

# FIG.6

# FIG.7

FREQUENCY

$N_1$

$N_2$

CORRECTED FEATURE

# FIG.8

200

R

$R_{S4}$

$R_{S1}$  $R_{S7}$

$R_{S2}$  $R_{S8}$

$R_{S3}$  $R_{S9}$

$R_{S6}$

$R_{S5}$

**FIG.9**

START

RECEIVE MEASUREMENT RESULT OF OBSERVATION TARGET — S1

AMPLIFY RECEIVED ECHO SIGNAL (STC CORRECTION) — S2

GENERATE B-MODE IMAGE DATA — S3

DISPLAY B-MODE IMAGE — S4

AMPLIFICATION CORRECTION — S5

FREQUENCY ANALYSIS — S6

CALCULATE PRE-CORRECTION FEATURE — S7

ATTENUATION CORRECTION ON PRE-CORRECTION FEATURE — S8

S9
DETERMINATION TARGET REGION IS VALID REGION? — NO

YES

$\alpha = \alpha_0$ — S10

ATTENUATION CORRECTION ON PRE-CORRECTION FEATURE USING ATTENUATION FACTOR CANDIDATE VALUE — S11

CALCULATE VARIANCE OF CORRECTED FEATURE OBTAINED BY ATTENUATION CORRECTION — S12

$\alpha = \alpha + \Delta\alpha$ — S13

S14
$\alpha > \alpha_{max}$? — NO

YES

SET ATTENUATION FACTOR CANDIDATE VALUE OF WHICH VARIANCE OF CORRECTED FEATURE IS SMALLEST AS OPTIMAL ATTENUATION FACTOR — S15

GENERATE FEATURE IMAGE DATA ON THE BASIS OF OPTIMAL ATTENUATION FACTOR — S16

S17
CALCULATE CORRECTED FEATURE USING PREDETERMINED ATTENUATION FACTOR

S18
GENERATE FEATURE IMAGE DATA

S19
SUBSEQUENT DETERMINATION TARGET REGION IS PRESENT?

YES

NO

DISPLAY FEATURE IMAGE — S20

END

# FIG.10

```
        ┌──────────────────┐
        │    FREQUENCY     │
        │     ANALYSIS     │
        └──────────────────┘
                 │
        ┌──────────────────┐
        │      k=k₀        │───S21
        └──────────────────┘
                 │
        ┌──────────────────┐
        │   Z^(k)=Z^(k)₀    │───S22
        └──────────────────┘
                 │
        ┌──────────────────────────┐
        │ ACQUIRE SAMPLE DATA GROUP │───S23
        └──────────────────────────┘
                 │
        ┌──────────────────────────┐
        │   APPLY WINDOW FUNCTION    │───S24
        └──────────────────────────┘
                 │
```

$k = k_0$ — S21

$Z^{(k)} = Z^{(k)}_0$ — S22

ACQUIRE SAMPLE DATA GROUP — S23

APPLY WINDOW FUNCTION — S24

S25 — SAMPLE DATA GROUP IS NORMAL? — NO

S26 — INSERT ZERO DATA CORRESPONDING TO SHORTAGE

YES

FFT PROCESSING — S27

$Z^{(k)} = Z^{(k)} + D$ — S28

S29 — $Z^{(k)} > Z^{(k)}_{max}$? — NO

YES

$k = k+1$ — S30

S31 — $k > k_{max}$? — NO

YES

RETURN

# FIG.11

# FIG.12

## INTERNATIONAL SEARCH REPORT

| International application No. |
|---|
| PCT/JP2016/084003 |

**A.   CLASSIFICATION OF SUBJECT MATTER**
*A61B8/14*(2006.01)i, *A61B8/12*(2006.01)i

According to International Patent Classification (IPC) or to both national classification and IPC

**B.   FIELDS SEARCHED**

Minimum documentation searched (classification system followed by classification symbols)
A61B8/14, A61B8/12

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| A | WO 2014/192954 A1   (Olympus Medical Systems Corp.), 04 December 2014 (04.12.2014), paragraph [0006] & JP 5659324 B1        & US 2015/0178919 A1 paragraph [0006] & EP 3005945 A1        & CN 104582584 A | 1–14 |
| A | WO 2012/133878 A1   (Olympus Medical Systems Corp.), 04 October 2012 (04.10.2012), paragraphs [0006], [0049], [0055] & JP 5114609 B2        & US 2013/0113938 A1 paragraphs [0006], [0061], [0067] & EP 2591729 A1        & EP 2842498 A1 & CN 102958447 A | 1–14 |

☒  Further documents are listed in the continuation of Box C.          ☐  See patent family annex.

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30 January 2017 (30.01.17) | 07 February 2017 (07.02.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2016/084003

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2012/063928 A1  (Olympus Medical Systems Corp.),<br>18 May 2012 (18.05.2012),<br>paragraphs [0030], [0078], [0079]<br>& JP 2013-166059 A      & US 2013/0035594 A1<br>paragraphs [0037], [0086], [0087]<br>& EP 2599440 A1          & CN 103200876 A | 1-14 |

Form PCT/ISA/210 (continuation of second sheet) (January 2015)

**EP 3 384 854 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013005876 A